**Europäisches Patentamt**

**European Patent Office**

**Office européen des brevets**

(19)

(11) Veröffentlichungsnummer : **0 244 664 B1**

(12) **EUROPÄISCHE PATENTSCHRIFT**

(45) Veröffentlichungstag der Patentschrift :
**05.06.91 Patentblatt 91/23**

(51) Int. Cl.⁵ : **G01N 3/42**

(21) Anmeldenummer : **87105350.0**

(22) Anmeldetag : **10.04.87**

(54) **Elektronische Messuhr für Härteprüfer.**

(30) Priorität : **09.05.86 DE 3615696**

(43) Veröffentlichungstag der Anmeldung :
**11.11.87 Patentblatt 87/46**

(45) Bekanntmachung des Hinweises auf die
Patenterteilung :
**05.06.91 Patentblatt 91/23**

(84) Benannte Vertragsstaaten :
**AT BE CH DE ES FR GB GR IT LI LU NL SE**

(56) Entgegenhaltungen :
**DE-A- 2 513 085**
**DE-C- 803 378**
**FR-A- 2 368 704**
**US-A- 3 295 365**
**US-A- 3 657 921**
**US-A- 4 036 048**

(73) Patentinhaber : **Kiffe, Horst-Gregor, Dipl.- Ing.**
**Vogelbeerweg 19**
**W-7730 Villingen-Schwenningen (DE)**

(72) Erfinder : **Kiffe, Horst-Gregor, Dipl.- Ing.**
**Vogelbeerweg 19**
**W-7730 Villingen-Schwenningen (DE)**

(74) Vertreter : **Patentanwälte Dipl.-Ing. Klaus**
**Westphal Dr. rer. nat. Bernd Mussgnug Dr.**
**rer.nat. Otto Buchner**
**Waldstrasse 33**
**W-7730 VS-Villingen-Schwenningen (DE)**

**Beschreibung**

Die Erfindung betrifft eine elektronische Digital-Meßuhr nach dem Oberbegriff des Anspruchs 1.

Eine derartige Meßuhr ist aus der US-A 4 036 048 bekannt, bei der ein großer mechanischer und elektronischer Aufwand zur Erhöhung der Meßgenauigkeit und Ausschaltung von Meßfehlern getrieben ist.

Die Anzeige von Meßuhren mit mechanischen Getrieben sind demgegenüber grundsätzlich infolge der auftretenden parasitären Reibungskräfte sowie des Zahn- und Lagerspiels im Getriebe ungenau. Außerdem ist eine datentechnische Weiterverarbeitung der Meßwerte bei Verwendung mechanischer Meßuhren problematisch bzw. nicht möglich. Es ist aber nicht nur ein herstellungsmäßiger, sondern auch ein großer Montageaufwand erforderlich, um ein bereits vorhandenes mechanisches Gerät mit einem elektronischen Meßsystem zu versehen.

Es besteht das Bedürfnis, die große Anzahl von seit Jahren im Einsatz befindlichen Härtemeßgeräten zu modernisieren und auch dokumentationsfähig zu machen, ohne einen derart großen Herstellungs- und Montageaufwand zu betreiben. Zur Erzielung einer guten Meßgenauigkeit ist es außer der bei bekannten Meßuhren verwirklichten Verdrehsicherung des Taststiftes und somit auch der Meßstange wichtig, daß der Steuerkern im Zentrum des Spulenkerns exakt positioniert wird. Ein aus mangelhafter Positionierung resultierender Fehler könnte dadurch unwirksam gemacht werden, daß man den Innendurchmesser der Meßspule so groß macht, daß Verdrehspiel-, Abstands- und Führungsfehler ohne Einfluß bleiben. Das würde jedoch wiederum auf Kosten der Baugröße der gesamten Meßuhr gehen.

Der Erfindung liegt daher die Aufgabe zugrunde, eine elektronische Digital-Meßuhr für Härteprüfer zu schaffen, die den Baumaßen einer mechanischen Meßuhr entspricht und die deswegen einen direkten Austausch der mechanischen Meßuhr gegen die elektronische Meßuhr mit geringstmöglichem Montageaufwand gestattet, wobei eine hervorragende Meßgenauigkeit erzielt werden soll.

Diese Aufgabe wird bei einer Meßuhr der eingangs genannten Art erfindungsgemäß durch die kennzeichnenden Merkmale des Anspruchs 1 gelöst.

Zweckmäßigerweise wird dabei zwischen Taststift und Meßstange eine kardanische oder halbkardanische Kopplung vorgesehen, welche eine exakte Führung der Meßstange in Längsrichtung des Taststiftes und im übrigen eine freie Beweglichkeit der Meßstange gewährleistet. Die Meßstange kann dann ihrerseits exakt in der Mitte der Meßspule geführt werden, und die auftretenden mechanischen Fehler werden von der Kopplung aufgenommen.

Bei einer zweckmäßigen Ausgestaltung der Erfindung gemäß Anspruch 2 besteht die Meßstange aus Keramikmaterial und der als Ferritkern bestehende Steuerkern ist in das Keramikmaterial eingebettet.

Die exakte Führung der Meßstange mit dem Steuerkern in der Meßspule wird gemäß Anspruch 3 vorteilhafterweise dadurch erreicht, daß der Hohlraum der Meßspule als genaue Linearführung für die im Durchmesser der lichten Weite der Linearführung genau angepaßte Meßstange mit Steuerkern ausgebildet ist. Es werden somit die unvermeidbaren Fertigungstoleranzen der Meßuhr eliminiert, andererseits die Verschiebung des Taststiftes exakt auf den Steuerkern übertragen. Durch das vollständige Ausfüllen des Innenquerschnitts der Meßspule durch den Steuerkern wird insbesondere der Wirkungsgrad des Meßsystems gesteigert, indem der magnetische Luftspalt entscheidend verkürzt und dadurch elektrische Verluste im Meßwandler vermieden werden. Durch die exakte Führung des Steuerkerns in der Meßspule wird ebenfalls die Meßgenauigkeit stark erhöht, weil weder das Verdrehspiel des Taststiftes noch Abstands- und Führungsfehler durch Herstellungstoleranzen einen Einfluß auf die Lage des Steuerkerns ausüben können. Trotz des geringen zur Verfügung stehenden Raumes kann eine hohe Linearität des Meßsystems mit außerordentlich geringen Toleranzen erzielt werden.

Besonders zweckmäßige Ausgestaltungen der halbkardanischen Kopplung zwischen Taststift und Meßstange sind in den Unteransprüchen 4 bis 7 unter Schutz gestellt.

Anhand der Figuren werden Ausführungsbeispiele der Erfindung näher erläutert. In sämtlichen Figuren sind nur die Taststift, Ausleger, Verbindungsglied und Meßspule umfassenden erfindungswesentlichen Teile der Meßuhr dargestellt. Es zeigt

Fig. 1 eine Seitenansicht der erfindungswesentlichen Teile einer ersten Ausführungsform,

Fig. 2 einen Schnitt längs der Linie II-II in Fig. 1,

Fig. 3 einen Schnitt längs der Linie III-III in Fig. 1,

Fig. 4 eine Seitenansicht der erfindungswesentlichen Teile einer zweiten Ausführungsform der Meßuhr,

Fig. 5 einen Schnitt längs der Linie V-V in Fig. 4, wobei die verbindende Klammer und der auf der Meßstange sitzende Ring weggelassen sind,

Fig. 6 eine Seitenansicht der erfindungswesentlichen Teile einer dritten Ausführungsform der Meßuhr,

Fig. 7 einen Schnitt längs der Linie VII-VII in Fig. 6,

Fig. 8 eine Seitenansicht der erfindungswesentlichen Teile einer vierten Ausführungsform der Meßuhr, wobei das Gehäuse der Meßuhr eingezeichnet ist, um den im Innern der Meßuhr zur Verfügung stehenden gedrängten Raum zu veranschaulichen, und

Fig. 9 einen Schnitt längs der Linie IX-IX in Fig. 8.

Die Wirkungsweise eines Rockwell-Härteprüfers wird als bekannt vorausgesetzt und von den Teilen dieses Geräts wird daher nur ein Abschnitt des der Prüfbelastung unterworfenen Taststiftes 10 dargestellt. Im Taststift 10 ist ein seitlich vorstehender Sicherungsstift 12 verankert, der in einer in Längsrichtung des Taststiftes 10 verlaufenden Nut 14 einer Führungsschiene 16 gleitend verschiebbar geführt ist. Dadurch ergibt sich eine Verdrehsicherung des Taststiftes 10 mit geringem Spiel.

Erfindungsgemäß ist an einer anderen Stelle des Taststiftes ein erster Abschnitt 18 eines senkrecht abstehenden Auslegers verankert, nahe dessen Ende eine Scheibe 20 befestigt ist, die eine radiale Schneide 22 aufweist. Auf der Schneide 22 liegt ein zweiter Abschnitt 24 des Auslegers auf, der parallel zum ersten Abschnitt 18 verläuft und diesen überlappt. Im überlappenden Bereich sind die beiden Abschnitte 18 und 24 des Auslegers durch einen elastischen O-Ring 26 kraftschlüssig zusammengehalten.

Am äußeren Ende des zweiten Auslegerabschnittes 24 ist eine Meßstange 28 aus keramischem Material nahe ihrem Ende derart befestigt, daß sie sich parallel zum Taststift 10 erstreckt. Die Meßstange durchsetzt den Hohlraum 30 einer Meßspule 32 und trägt in diesem Bereich einen als Ferritkern ausgebildeten induktiven Steuerkern 34. Der Hohlraum 30 ist als exakte Linearführung für die Meßstange 28 ausgebildet, wobei der Durchmesser der Meßstange der lichten Weite der Linearführung genau angepaßt ist.

Eine Längsverschiebung des Taststiftes 10 wird über die Schneide 22 exakt und spielfrei auf den Steuerkern 34 übertragen, wobei eventuell auftretende Spiele und Fertigungstoleranzen von der halbkardanischen, elastischen Kopplung 20, 22, 26 aufgenommen werden.

Bei den folgenden Ausführungsbeispielen werden für gleiche oder entsprechende Teile die gleichen Bezugszeichen verwendet wie bei der ersten Ausführungsform.

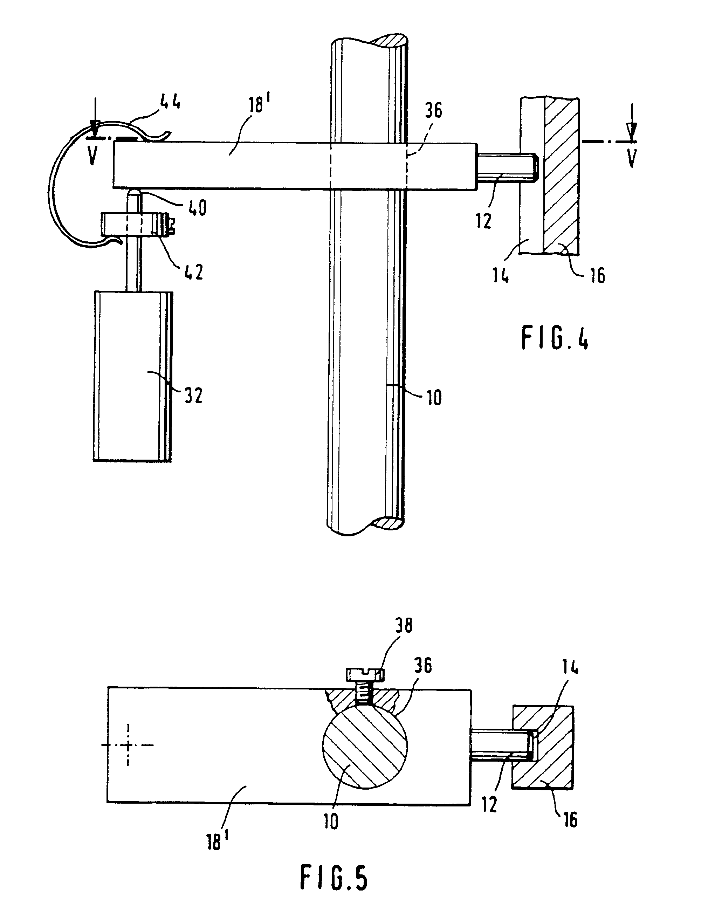

Bei der in den Fig. 4 und 5 dargestellten Ausführungsform ist der Sicherungsstift 12 nicht unmittelbar am Taststift 10 befestigt, sondern ragt einstückig aus dem Ende eines einteiligen Auslegers 18' vor. Der Ausleger 18' weist eine Querbohrung 36 auf, in der der Taststift 10 mittels einer Schraube 38 festgelegt ist.

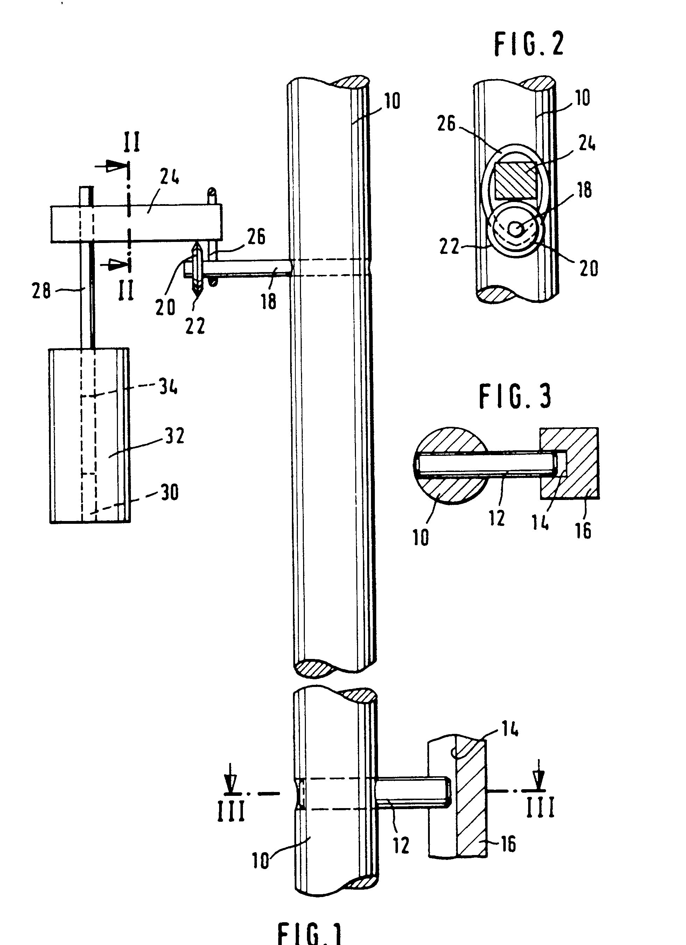

Das entgegengesetzte Ende des Auslegers 18' liegt auf einer abgerundeten Kuppe 40 am Ende der Meßstange 28 auf. Die Meßspule 32 und die in ihr vorgesehenen Teile sind ebenso ausgebildet wie bei der in Fig. 1 dargestellten Ausführungsform und müssen daher hier nicht nochmals dargestellt werden.

In dem außerhalb der Meßspule 32 gelegenen Abschnitt trägt die Meßstange einen Anschlagring 42. Eine elastische Klammer 44 hält Anschlagring 42 und Ausleger 18' kraftschlüssig zusammen.

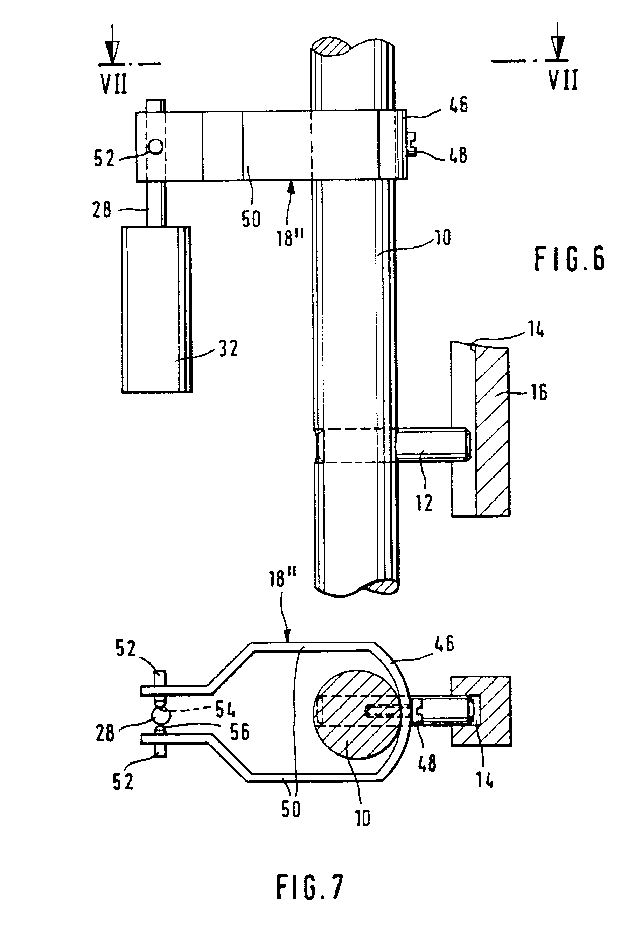

Auch bei der in den Fig. 6 und 7 dargestellten Ausführungsform ist der Ausleger 18'' einstückig in Form eines aus Federstahl oder Material mit ähnlichen Biegeeigenschaften bestehenden zur Längsrichtung des Taststiftes 10 hochkant angeordneten Bandes ausgebildet. Das Federstahlband 18'' weist an der von der Meßstange 28 abgelegenen Seite des Taststiftes 10 einen gekrümmten Abschnitt 46 auf, dessen Krümmung etwas geringer ist als die Krümmung des Außenumfanges des Taststiftes 10. Der Abschnitt 46 liegt daher nur linienförmig am Taststift 10 an und ist in diesem Bereich durch eine Schraube 48 am Taststift 10 befestigt. Die beiden in Richtung zur Meßstange 28 hinweisenden Arme 50 des Auslegers 18'' tragen nahe ihren gegenüberliegenden Enden je einen Stift 52 mit einer in je eine Vertiefung 54 der Meßstange 28 durch die federnde Wirkung der Arme 50 eingedrückten Spitze 56. Da die Biegesteifigkeit des Auslegers 18'' durch die Anordnung hochkant zur Längsrichtung des Taststiftes 10 quer zu dieser Längsrichtung sehr groß ist und andererseits eine kraftschlüssige Verbindung der Auslegerarme 50 mit der Meßstange 28 durch das Eindringen 56 in die Vertiefungen 54 erzielt wird, ergibt sich bei Verschiebungen des Taststiftes 10 in seiner Längsrichtung eine äußerst exakte Mitführung der Meßstange 28. Andererseits wird ein eventuelles Verdrehspiel des Taststiftes 10 und ein auf Fertigungstoleranzen beruhender Abstands- und Führungsfehler durch die elastische Verbindung zwischen Taststift 10 und Meßstange 28 ausgeglichen und bleibt ohne Einfluß auf die Messung.

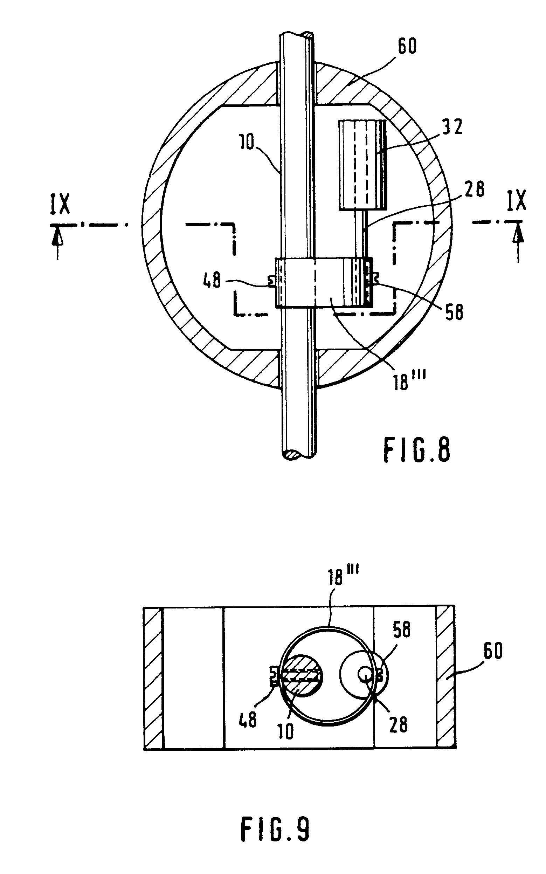

Bei der in kleinerem Maßstab gezeichneten Ausführungsform gemäß Fig. 8 und 9 ist als einstückiger Ausleger 18''' ebenfalls ein hochkant zur Längserstreckung des Taststiftes 10 angeordnetes, kreisrundes in sich geschlossenes Federstahlband vorgesehen. Es liegt jeweils linienförmig an der voneinander wegweisenden Seite des Taststiftes 10 und der Meßstange 28 an und ist in diesen Bereichen jeweils durch eine Schraube 48 bzw. 58 am Taststift 10 bzw. an der Meßstange 28 befestigt. Diese Ausführungsform ist, wie ersichtlich, hauptsächlich zum Ausgleich von Verdrehspiel und Abstandstoleranz geeignet, während Führungsfehler hierdurch nicht ausgeglichen werden können. Bei sehr exakter Führung der Meßstange 28 ist dies jedoch in vielen Fällen nicht erforderlich, so daß diese vereinfachte Ausführungsform ausreicht.

Zusätzlich ist in den Fig. 8 und 9 ein in die gesamte Meßuhr mit den erfindungswesentlichen Teilen und den nicht dargestellten übrigen Teilen einer elektronischen Digital-Meßuhr umgebendes Gehäuse 60 dargestellt, dessen Größe weitgehend bisher üblichen und für diesen Zweck verwendeten mechanischen Meßuhren entspricht. Die nicht dargestellten elektronischen Teile der Meßuhr sind in an sich bekannter Weise mit der Meßspule 32 verbunden. Aus dieser Darstellung des Gehäuses 60 ist ersichtlich, daß jederzeit eine vorhandene mechanische Meßuhr durch eine erfindungsgemäße elektronische Digital-Meßuhr ersetzt werden kann, wobei kein zusätzlicher Platzbedarf geschaffen werden muß und der Montageaufwand sich in engen Grenzen hält. So kann jederzeit ein vorhandenes Härteprüfgerät mit einer erfindungsgemäßen elektronischen Meßuhr nachgerüstet werden. Die Teile 12, 14, 16 zur Verdrehsicherung des Taststiftes sind bei dieser Ausführungsform nicht dargestellt. Ferner ist zu dieser Ausführungsform zu vermerken, daß die Meßstange 28 vom Ausleger 18‴ nach oben steht, was bei den anderen Ausführungsformen ohne Beeinträchtigung der Wirkungsweise ebenfalls ohne weiteres möglich wäre.

**Ansprüche**

1. Elektronische Digital-Meßuhr für einen insbesondere nach dem Rockwell-Verfahren arbeitenden Härteprüfer, mit einem die Stellung des Taststiftes (10) des Härteprüfers abtastenden Meßwertgeber, wobei am Taststift (10) ein Ausleger (18, 18′, 18″, 18‴) angebracht ist, der mit einer parallel zum Taststift (10) angeordneten Meßstange (28) gekoppelt ist, und wobei an der Meßstange (28) ein induktiver Steuerkern (34) befestigt ist, der den Hohlraum (30) einer stationären Meßspule (32) durchsetzt, dadurch gekennzeichnet, daß die Kopplung des Auslegers (18, 18′, 18″, 18‴) mit der Meßstange (28) über ein elastisches Verbindungsglied (20, 22, 26 ; 40, 42, 44; 18″, 52, 54, 56 ; 18‴) eine exakte Führung der Meßstange in Längsrichtung des Taststiftes und im übrigen eine freie Beweglichkeit der Meßstange ermöglicht.

2. Meßuhr nach Anspruch 1, dadurch gekennzeichnet, daß die Meßstange (28) aus Keramikmaterial besteht und der Steuerkern (34) als in das Keramikmaterial eingebetteter Ferritkern ausgebildet ist.

3. Meßuhr nach Anspruch 1 oder 2, dadurch gekennzeichnet, daß der Hohlraum (30) der Meßspule (32) als genaue Linearführung für die im Durchmesser der lichten Weite der Linearführung genau angepaßte Meßstange (28) mit Steuerkern (34) ausgebildet ist.

4. Meßuhr nach einem der vorangehenden Ansprüche, dadurch gekennzeichnet, daß der Ausleger aus zwei sich überlappenden, getrennten Abschnitten (18, 24) besteht, wobei der erste Abschnitt (18) am Taststift (10) und der zweite Abschnitt (24) an der Meßstange (28) befestigt ist, und daß der erste Abschnitt (18) am zweiten Abschnitt (24) oder umgekehrt mittels eines in Längsrichtung des Taststiftes (10) wirksamen Schneiden- oder Spitzenlagers (22) abgestützt ist, wobei die beschnitte (18, 24) mittels eines elastischen Ringes (26) zusammengehalten sind.

5. Meßuhr nach einem der Ansprüche 1 bis 3, dadurch gekennzeichnet, daß die Meßstange (28) mit einem abgerundeten Ende (40) oder einer Spitze am Ausleger (18′) anliegt, daß die Meßstange (28) einen Anschlagring (42) trägt und daß Anschlagring (42) und Ausleger (18′) durch eine elastische Klammer (44) zusammegehalten sind.

6. Meßuhr nach einem der Ansprüche 1 bis 3, dadurch gekennzeichnet, daß der Ausleger (18″) als parallel zum Taststift (10) hochkant stehendes, doppelarmiges Federstahlband (46, 50) ausgebildet ist, das am Taststift (10) befestigt ist und dessen beide Arme (50) jeweils mittels eines Spitzenlagers (54, 56) an der Meßstange (28) gelagert sind.

7. Meßuhr nach einem der Ansprüche 1 bis 3, dadurch gekennzeichnet, daß der Ausleger (18″) als etwa kreisrundes, geschlossenes Federstahlband ausgebildet ist, das an den voneinander wegweisenden Seiten des Taststiftes (10) und der Meßstange (28) befestigt ist.

**Claims**

1. An electronic digital indicating device for a hardness tester, especially one performing a Rockwell hardness test, comprising a sensor detecting the position of the feeler rod (10) of the hardness tester, wherein a projecting arm (18, 18′, 18″, 18‴) is connected to the feeler rod (10), which arm is coupled to a measurement rod (28) arranged parallel to the feeler rod (10), and wherein an inductive control core (34) is fastened to the measurement rod (28) which passes into the hollow space (30) in a stationary measurement coil (32), characterized in that the coupling of the projecting arm (18, 18′, 18″, 18‴) with the measurement rod (28) by means of an elastic connection element (20, 22, 26 ; 40, 42, 44 ; 18″, 52, 54, 56; 18‴) enables exact guidance of the measurement rod in the longitudinal direction of the feeler rod but free movement of the measurement rod otherwise.

2. An indicating device according to claim 1, characterized in that the measurement rod (28) is made of ceramic material and the control core (34) is formed as a ferrite core embedded in the ceramic material.

3. An indicating device according to claim 1 or claim 2, characterized in that the hollow space (30) in the measurement coil (32) is formed as an exact linear guide for the measurement rod (28), comprising the control core (34), which is exactly adapted in diameter to the clear width inside the linear guide.

4. An indicating device according to any one of the preceding claims, characterized in that the projecting arm consists of two separate, overlapping sections (18, 24), where the first section (18) is fastened to the feeler rod (10) and the second section (24) is fastened to the measurement rod (28), and the first section (18) is supported on the second section (24), or conversely, by means of a knife bearing or point bearing (22) operating in the longitudinal direction of the feeler rod (10), and the sections (18, 24) are held together by an elastic ring (26).

5. An indicating device according to any one of claims 1 to 3, characterized in that the measurement rod (28) bears by means of a rounded end (40) or a tip on the projecting arm (18'), the measurement rod (28) carries a bearing ring (42), and the bearing ring (42) and the projecting arm (18') are fastened together by a resilient clamp (44).

6. An indicating device according to any one of claims 1 to 3, characterized in that the projecting arm (18'') is formed as a two-armed spring steel band (46, 50), standing upright parallel to the feeler rod (10), which is fastened to the feeler rod (10) the two arms (50) of which each bear by means of a point bearing (54, 56) on the measurement rod (28).

7. An indicating device according to any one of claims 1 to 3, characterized in that the projecting arm (18''') is formed as an approximately circular closed spring steel band which is fastened to the sides of the feeler rod (10) and of the measurement rod (28) which face away from each other.


**Revendications**

1. Comparateur-amplificateur électronique numérique pour un duromètre fonctionnant notamment d'après la méthode Rockwell, comprenant un capteur de mesure explorant la position du palpeur (10) du duromètre, le palpeur (10) étant muni d'un bras (18, 18', 18'', 18''') qui est couplé avec une tige de mesure (28) disposée parallèlement au palpeur (10), et la tige de mesure (28) portant un noyau de commande inductif (34) qui traverse la cavité (30) d'une bobine détectrice stationnaire (32), **caractérisé en ce** que le couplage du bras (18, 18', 18'', 18''') avec la tige de mesure (28) par l'intermédiaire d'un élément de raccordement élastique (20, 22, 26 ; 40, 42, 44 ; 18'', 52, 54, 56 ; 18''') permet un guidage exact de la tige de mesure dans le sens longitudinal du palpeur et, par ailleurs, une libre mobilité de la tige de mesure.

2. Comparateur-amplificateur selon la revendication 1, caractérisé en ce que la tige de mesure (28) est réalisée en céramique et que le noyau de commande (34) est conformé en noyau de ferrite encastré dans de la céramique.

3. Comparateur-amplificateur selon l'une des revendications 1 ou 2, caractérisé en ce que la cavité (30) de la bobine détectrice (32) constitue un guidage linéaire exact pour la tige de mesure (28) avec le noyau de commande (34) dont le diamètre est adapté avec précision au diamètre intérieur du guidage linéaire.

4. Comparateur-amplificateur selon l'une des revendications précédentes, caractérisé en ce que le bras se compose de deux sections (18, 24) séparées en recouvrement, la première section (18) étant fixée sur le palpeur (10) et la seconde section (24) étant montée sur la tige de mesure (28) ; et que la section (18) prend appui sur la seconde section (24) ou inversement au moyen d'un support à couteau ou à pivot (22) agissant dans le sens longitudinal du palpeur (10), les sections (18, 24) étant maintenues ensemble au moyen d'une bague élastique (26).

5. Comparateur-amplificateur selon l'une des revendications 1 à 3, caractérisé en ce que la tige de mesure (28) est appliquée avec une extrémité arrondie (40) ou une pointe contre le bras (18') ; que la tige de mesure (28) porte une bague de butée (42) et que la bague de butée (42) et le bras (18') sont maintenus ensemble par une bride de fixation élastique (44).

6. Comparateur-amplificateur selon l'une des revendications 1 à 3, caractérisé en ce que le bras (18'') est réalisé sous la forme d'une bande d'acier à ressorts (46, 50) à deux bras qui est placée parallèlement au palpeur (10) en position debout et fixée sur le palpeur (10) et dont les deux bras (50) sont montés chacun sur la tige de mesure (28) au moyen d'un support à pivot (54, 56).

7. Comparateur-amplificateur selon l'une des revendications 1 à 3, caractérisé en ce que le bras (18''') est réalisé sous la forme d'une bande d'acier à ressorts fermée et sensiblement circulaire qui est fixée sur des côtés opposés du palpeur (10) et de la tige de mesure (28).

FIG. 2

FIG. 3

FIG.1

FIG.4

FIG.5

FIG.6

FIG.7

FIG.8

FIG.9